# EUROPEAN PATENT SPECIFICATION

(11) **EP 0 493 387 B1**
(45) Date of publication and mention of the grant of the patent: **20.10.1993**
(21) Application number: 90911058.7
(22) Date of filing: 18.07.1990
(51) Int. Cl.: A61J 1/00, A61L 25/00, F25C 1/10, A61M 5/178, A61M 11/00, B65D 81/18

(54) **A MEDICAL DISPENSING SYSTEM FOR TISSUE ADHESIVE COMPONENTS**
MEDIZINISCHES SPENDESYSTEM FÜR GEWEBEKLEBSTOFFKOMPONENTEN
SYSTEME DE DISTRIBUTION MEDICALE DE COMPOSANTS ADHESIFS TISSULAIRES

(30) Priority: 10.08.1989 US 391869
(43) Date of publication of application: 08.07.1992
(73) Proprietor: W.L. GORE & ASSOCIATES, INC., Newark, Delaware 19714-9206 (US)
(72) Inventor: HULL, John, Richard, Flagstaff, AZ 86004 (US); SHARBER, Norman, Joseph, Flagstaff, AZ 86001 (US)
(74) Representative: Klunker . Schmitt-Nilson . Hirsch
(86) International application number: US9004043
(87) International publication number: WO9101711

(56) References cited:
- FR-A- 1 565 256
- FR-A- 2 574 054
- GB-A- 1 103 534
- GB-A- 1 441 481
- GB-A- 2 042 556
- US-A- 2 655 007
- US-A- 3 240 328
- US-A- 3 640 081
- US-A- 4 139 992
- Adv. Biomater., volume 3, 1982, H. Redl et al.: "Background and methods of "fibrin sealing", pages 669 - 676 see page 669, line 1 - page 670, line 4; page 672, lines 17 - 36; figure 6

## Description

### FIELD OF THE INVENTION

This invention provides a medical dispensing system for making tissue adhesive components quickly available for surgical use and a process for preparing same.

### BACKGROUND OF THE INVENTION

Uncontrollable bleeding from traumatic or surgically induced wounds can greatly increase the time needed to surgically treat wounds and can in some cases be life threatening. A variety of hemostatic agents have been developed to decrease wound bleeding. There are two main types: 1) clotting stimulants that stimulate the natural blood clotting mechanisms or provide an excess of a natural clotting component (eg. thrombin), relying on the patient's own blood to provide the other components (eg. fibrinogen), and 2) self-polymerizing tissue adhesives that do not rely on the patient's blood or blood components to assist in hemostasis. The first type includes oxidized cellulose, collagen sponges, gels and powders, cryoprecipitate from cell-free blood plasma, or its major active coagulation ingredients fibrinogen or thrombin. The second type of hemostatic agents include single and multi-component tissue adhesives that differ from clotting stimulants in that they are in themselves self-polymerizing and do not rely on the patient's blood or blood components to play an active role in hemostasis. Such adhesives can be used to adhere tissue to tissue. Tissue adhesives are considered to be more efficacious hemostatic agents than the blood coagulation stimulants.

The earliest tissue adhesives were based on cyanoacrylates, however, tissue toxicity problems with these adhesives has prevented their widespread use.

"Fibrin glue" is currently the most widely used tissue adhesive for hemostasis. Fibrin glue has two major components: fibrinogen and thrombin. Other components such as calcium, calcium salts, Factor XIII and the like may be added if necessary. The components must be kept separate until use because as soon as they are combined, thrombin will rapidly catalyze the conversion of fibrinogen to fibrin monomers which then quickly polymerize into a fibrin network. To control bleeding, the separate components of fibrin glue are simultaneously applied to the desired site with two separate syringes or with two separate syringes in a common syringe holder. They can also be applied in the form of a spray to stop bleeding from a diffuse source, such as a highly vascularized tissue bed.

There are two common methods of preparing fibrin glue: 1) A cryoprecipitate is obtained from the patient's own cell-free blood plasma and mixed during application with a commercially available bovine thrombin. A disadvantage of this method is that the amount of fibrinogen and other necessary ingredients in the cryoprecipitate varies widely from donor to donor, hence the fibrin glue will be of unknown quality. Another disadvantage is that the blood must be withdrawn from the patient in advance, which may increase the likelihood of the patient requiring a blood transfusion. 2) A commercially available fibrin glue kit" (Tisseal®, Immuno, A.G., Vienna, Austria), described in the article "Background and methods of fibrin sealing", Redl et al, Biomaterials 1980, pp 669-676, overcomes the quality variability problem. This kit provides a lyophilized form of fibrinogen and thrombin, along with the necessary fluids for reconstitution. One of the components of the kit, fibrinogen, is relatively insoluble. A magnetic stirrer is usually provided to help dissolve the fibrinogen, but the dissolution process can require up to 20 minutes. Very substantial blood loss can occur during that time. This time constraint essentially requires that a surgeon decide beforehand that fibrin glue will be necessary.

It would be desirable to provide means for preparing tissue adhesives such as fibrin glue easily and quickly, so that the preparation will be available for use by the surgeon in a relatively short period of time, for example, within approximately 5 minutes. With such short preparation times, a surgeon does not have to determine in advance that a tissue adhesive will be required.

Tissue adhesives and tissue adhesive components are hereinafter collectively referred to as tissue adhesive components.

### SUMMARY OF THE INVENTION

This invention provides a novel medical dispensing system for making tissue adhesive components quickly available for surgical use comprising:
(a) one or more closed containers, each of the containers having at least one interior surface;
(b) a thin frozen coating of a tissue adhesive component on at least one interior surface of said closed containers.

The thin frozen coating of tissue adhesive component can be quickly thawed, mixed with another tissue adhesive component if necessary, and immediately applied to a bleeding wound.

The containers may include means for dispensing the tissue adhesive components, in the form of syringes, aerosol dispensers and the like.

### BRIEF DESCRIPTION OF THE DRAWINGS

- Figure 1: shows in longitudinal section a cylindrical container having inside a frozen tissue adhesive component in the form of a thin coating dispersed and frozen over the curved interior surface of the container.
- Figure 2: shows a double syringe suitable for mixing and applying two separate tissue adhesive components when in liquid states; each syringe is shown in longitudinal section, containing a frozen tissue adhesive component in the form of a thin coating dispersed and frozen over the curved interior surface of the container.
- Figure 3: shows in longitudinal section an aerosol dispensing container having inside a frozen tissue adhesive component in the form of a thin coating dispersed and frozen over the curved interior surface of the container.

### DETAILED DESCRIPTION OF THE INVENTION

It is the purpose of this invention to substantially reduce the amount of time presently required to make tissue adhesives available for surgical use. This is accomplished by placing a solution containing the desired tissue adhesive component or components in a container, closing the container and freezing the solution in the container. The solution can be a liquid or a colloid. The container is rapidly rotated around its axis while being frozen, thus coating at least one interior surface of the container with a thin coating of frozen tissue adhesive component. The tissue adhesive components are maintained frozen in the form of a thin coating over an interior surface of the container until needed. Thus, the container also serves as a storage device for the frozen tissue adhesive component. In use, the frozen contents of the container or containers are readily thawed, thus making the contents quickly available for application to a bleeding wound.

The advantages of this invention include the quick availability for use compared to the methods currently available. The methods currently available require preparation times on the order of twenty minutes. The present invention provides ready use of the solutions in times significantly less than twenty minutes, often requiring at most five minutes of time.

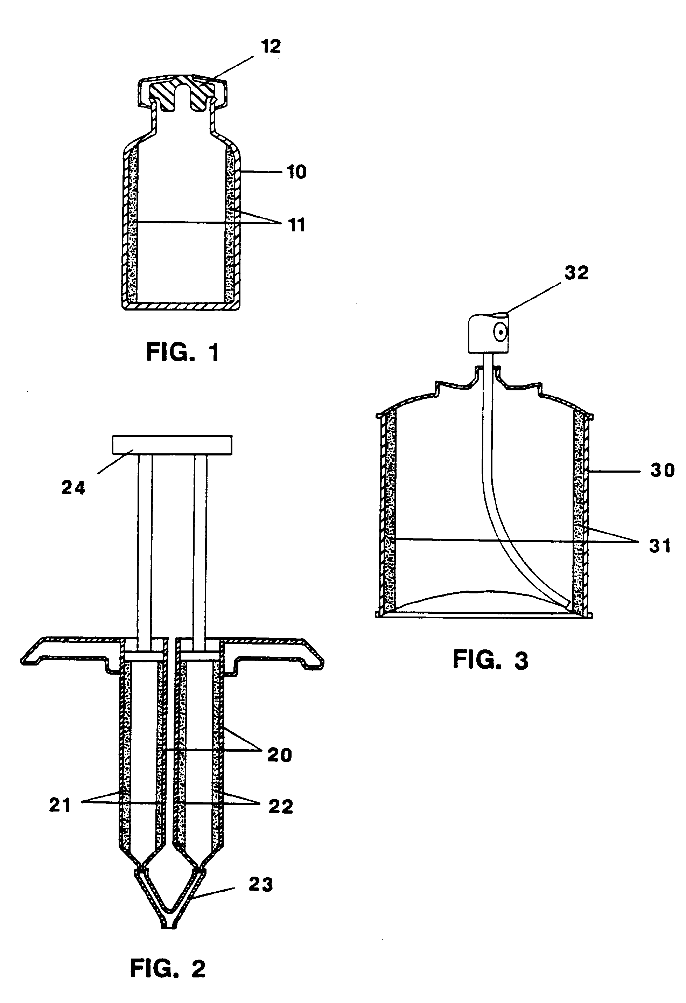

Figure 1 shows a longitudinal section of a medical dispensing system comprising a container (10) having inside a frozen tissue adhesive component (11) in the form of a thin coating dispersed and frozen over at least one interior surface of the container. This dispensing system was made by placing a solution containing a tissue adhesive component into the container, closing the container and then rotating the container around its axis at a speed high enough to cause the liquid tissue adhesive component to be dispersed in the form of a thin coating over the curved interior surface of the container. The contents of the container were then frozen while the container continued to be rotated.

The container (10) should most preferably be of cylindrical shape. Other shapes may be used if a thin enough coating is produced during spinning to subsequently allow thawing to be accomplished within the desired time. Container shapes other than cylindrical will result in a frozen coating of tissue adhesive component of non-uniform thickness which will subsequently increase the time required for thawing.

The time required for thawing the contents of the container is a function of the thickness of the thin coating of frozen tissue adhesive components, the thickness, shape and heat transfer characteristics of the material comprising the container and the method used for thawing the tissue adhesive component. A thin coating is therefore defined as a coating of thickness such that the coating may be thawed, that is made quickly available for surgical use, when heat is applied to the container by a suitable method.

The containers may be of any material such that the contents may be packaged sterile, that adequate physical protection is provided to the contents and that is able to withstand the temperature range necessary for freezing and thawing the contents of the containers. Some plastics, metals, and glass are among the materials suitable for this application. The material and the container wall thickness chosen should minimize the time necessary for thawing the thin frozen coating.

Any suitable method may be utilized to thaw the contents in order to make them quickly available for application, so long as the method chosen does not denature the protein components of the contents or otherwise adversely affect their adhesive characteristics. Both syringe and aerosol type containers that had been partially filled with tissue adhesive components-and frozen in the manner previously described have been successfully thawed within five minutes by immersion in 37°C water, exposure to radiant heat from an infrared lamp and simply by holding the container in a human hand. In the case of the heat lamp, the container was rotated along its longitudinal axis in front of the lamp while the surface temperature of the container was monitored with a thermocouple to ensure that the container temperature did not exceed 40°C. An alternate method of thawing would be to use a specially designed heating device that would conduct heat directly to the container while avoiding the possibility of overheating.

The contents of the container shown in Figure 1 may be dispensed after thawing by, for example, inserting the needle of a syringe through the rubber container cap (12) and withdrawing the contents. The syringe can then be used to apply the contents to the desired surgical site.

The containers can include means for dispensing the thawed tissue adhesive components. For example, the container may be a syringe. If it is necessary to mix two tissue adhesive components immediately prior to application to tissue, a double barreled mixing syringe (Fig. 2) with a single outlet (23) can be utilized in which each barrel of the syringe contains a different tissue adhesive component (21 and 22). After thawing, the separate components are mixed during application by applying pressure to the common handle (24) typically utilized with the pair of syringe plungers. The separate components (21 and 22) are thus simultaneously forced into the outlet tube (23) of the syringe assembly. Mixing may be made to occur either within the outlet tube (23) of the syringe assembly or at the tip of the outlet tube, according to the design of the outlet tube. Syringes for this type of application have been available for some time, however, they have not heretofore been utilized for the quick delivery of previously frozen tissue adhesive components.

If a double barreled mixing syringe is utilized for this application it is desirable to load, spin and freeze each syringe (20) individually before installing the syringes into the double barreled distribution device. This is due to the need to spin each syringe along its longitudinal axis during freezing in order to provide an even distribution of the fluid contents over the curved inner surface of each syringe.

The means for dispensing the previously frozen and thawed tissue adhesive components can also be an aerosol or atomized spray. Figure 3 shows a container (30) with an aerosol dispensing mechanism (32), the contents of the container being a thin frozen coating of tissue adhesive component (31). The containers may be pressurized with any suitable propellant so as to enable them to exhaust their contents as an aerosol, or they may be provided with a pump mechanism, preferably hand actuated, in order to deliver the contents in the form of an atomized spray. Either method is suitable when it is desired to uniformly distribute the container's contents over the surface of a surgical site. Where it is desired to mix two components during application, an application mechanism may be utilized that would simultaneously spray the separate contents of two or more containers over the desired surface.

Fibrinogen and thrombin, the basic tissue adhesive components comprising fibrin glue, are the most likely materials for use by the method of this invention. Additives such as calcium, calcium salts, Factor XIII and the like, or anti-fibrinolytic agents such as aprotinin and the like, may be included if necessary before freezing.

### EXAMPLE 1

A cylindrical 3 cc polypropylene syringe of about 0.35 inch (0.85 cm) inside diameter, 0.42 inch (1.0 cm) outside diameter and 1.9 inch (4.6 cm) inside length, containing about 1 cc of cell-free Human Plasma Cryoprecipitate AHF obtained from United Blood Services, Scottsdale, Arizona, was rotated at about 1770 rpm while lowering the container temperature until the contents were frozen. Freezing temperatures were attained by spraying the syringe with carbon dioxide gas. A similar syringe containing about 1 cc of bovine thrombin, obtained from ICN Immuno Biologicals, Lisle, Illinois, was prepared in a similar fashion. The curved interior surfaces of the syringes were found to be coated with a uniform thin frozen layer about 0.032 inches (0.077 cm) thick of content material. Both syringes were stored frozen at about -20°C until use approximately 15 hours later. The container and contents were then thawed in less than five minutes by holding the container in a human hand. The thawed contents were immediately utilized to retard bleeding at the anastomosis of a prosthetic vascular graft that had been implanted in a greyhound dog as a carotid interposition. Upon completion of the anastomosis, the contents were applied to the anastomosis and suture holes in a fashion similar to the normal application of fibrin glue. Hemostasis was achieved in the same time frame as would be expected using conventionally prepared fibrin glue.

### EXAMPLE 2

5cc of cell-free human plasma cryoprecipitate was placed into a first cylindrical glass aerosol container of about 1.20 inch (2.9 cm) inside diameter, 1.43 inch (3.46 cm) outside diameter and 1.75 inch (4.2 cm) inside length. 5cc of bovine thrombin was placed into a second cylindrical glass aerosol container of dimensions identical to the first container. Both the cell-free human plasma cryoprecipitate and the bovine thrombin were obtained from the sources described in Example 1. Each container was rotated at about 1600 rpm while subjected to freezing temperatures in the same manner as described in Example 1. The frozen contents coated the curved interior surface of the container to a uniform thickness of about 0.048 inches (0.12 cm) in each case. The containers were then charged with nitrogen gas as a propellant. After thawing by holding the container in a human hand for less than five minutes, the contents were sprayed simultaneously to retard bleeding in a surgical wound created in a spleen of a greyhound dog. Bleeding from wounds in the spleen and other similar highly vascularized tissue beds is especially difficult to control. It was found when applying the aerosol that the pressure of the propellant displaced the blood from the surface of the wound, hence allowing the fibrin glue to coagulate directly on the bleeding tissue. This was much more effective in stopping bleeding from such a wound than glue applied from a syringe, which typically results in fibrin glue forming on the surface of the exposed blood rather than on the surface of the wounded tissue. Syringe application may not prevent continued bleeding of this type of wound because fibrin glue applied by syringe will often wash away during bleeding.

## Claims

1. A medical dispensing system for making tissue adhesive components quickly available for surgical use comprising:
(a) one or more closed container (10,20,30), each of the closed containers having at least one interior surface;
(b) a thin frozen coating of a tissue adhesive component (11,21,22,31) on at least one interior surface of each of the closed containers;
wherein the tissue adhesive component remains in the form of a thin frozen coating during storage but is thawed immediately before dispensing for surgical use.

2. A medical dispensing system according to claim 1 wherein at least one of the tissue adhesive components comprises fibrinogen.

3. A medical dispensing system according to claim 1 wherein at least one of the tissue adhesive components comprises thrombin.

4. A medical dispensing system according to claim 1 wherein at least one of the tissue adhesive components comprises cell-free human blood cryoprecipitate.

5. A medical dispensing system according to claim 1 wherein the containers are of cylindrical form.

6. A medical dispensing system according to claim 1 wherein said medical dispensing system includes means for quickly thawing the frozen tissue adhesive components.

7. A medical dispensing system according to claim 1 wherein said containers include means for dispensing the tissue adhesive components after thawing.

8. A medical dispensing system according to claim 7 wherein said means for dispensing comprises a syringe (20).

9. A medical dispensing system according to claim 7 wherein said means for dispensing comprises an aerosol dispenser (30).

10. A process for preparing a medical dispensing system for making tissue adhesive components quickly available for surgical use comprising:
(a) placing one or more tissue adhesive components in liquid state into one or more containers (10,20,30), each container having at
(b) closing said containers;
(c) rotating said containers and tissue adhesive components at a rate fast enough to cause said tissue adhesive components to form a thin coating on at least one interior surface of each container; and
(d) freezing said tissue adhesive components during rotation so as to form thin coatings of frozen tissue adhesive components (11,21,22,31).

11. A process according to claim 10 wherein one of the tissue adhesive components is fibrinogen.

12. A process according to claim 10 wherein one of the tissue adhesive components is thrombin.

## Patentansprüche

1. Medizinisches Abgabe-System zur Bildung
von Gewebe-Klebstoff-Komponenten, welche für den chirurgischen Gebrauch schnell verfügbar sind, aufweisend:
(a) einen oder mehrere geschlossene Behälter (10, 20, 30), wobei ein jeder der geschlossenen Behälter mindestens eine innere Oberfläche aufweist;
(b) eine dünne gefrorene Schicht aus einer Gewebe-Klebstoff-Komponente (11, 21, 22, 31) auf mindestens einer inneren Oberfläche eines jeden der geschlossenen Behälter;
wobei die Gewebe-Klebstoff-Komponente in der Form einer dünnen gefrorenen Schicht während der Speicherung verbleibt, jedoch unmittelbar vor der Abgabe für den chirurgischen Gebrauch aufgetaut wird.

2. Medizinisches Abgabe-System nach Anspruch 1, bei welchem zumindest eine der Gewebe-Klebstoff-Komponenten Fibrinogen aufweist.

3. Medizinisches Abgabe-System nach Anspruch 1, bei welchem zumindest eine der Gewebe-Klebstoff-Komponenten Thrombin aufweist.

4. Medizinisches Abgabe-System nach Anspruch 1, bei welchem zumindest eine der Gewebe-Klebstoff-Komponenten Kryopräzipitat von zellfreiem menschlichen Blut aufweist.

5. Medizinisches Abgabe-System nach Anspruch 1, bei welchem die Behälter von zylindrischer Form sind.

6. Medizinisches Abgabe-System nach Anspruch 1, bei welchem das medizinische Abgabe-System Mittel zum raschen Auftauen der gefrorenen Gewebe-Klebstoff-Komponenten aufweist.

7. Medizinisches Abgabe-System nach Anspruch 1, bei welchem die genannten Behälter Mittel zum Abgeben der Gewebe-Klebstoff-Komponenten nach dem Auftauen aufweisen.

8. Medizinisches Abgabe-System nach Anspruch 7, bei welchem die genannten Mittel zum Abgeben eine Spritze (20) aufweisen.

9. Medizinisches Abgabe-System nach Anspruch 7, bei welchem die genannten Mittel zum Abgeben eine Aerosol-Abgabevorrichtung (30) aufweisen.

10. Verfahren zum Herstellen eines medizinischen Abgabe-Systems zum Bilden von Gewebe-Klebstoff-Komponenten, welche für den chirurgischen Gebrauch rasch verfügbar sind, aufweisend:
(a) Einbringen von einer oder von mehreren Gewebe-Klebstoff-Komponenten in flüssigem Zustand in einen oder mehrere Behälter (10, 20, 30), wobei ein jeder Behälter zumindest eine innere Oberfläche aufweist;
(b) Verschliessen der genannten Behälter;
(c) Rotieren der genannten Behälter und der Gewebe-Klebstoff-Komponenten mit einer Geschwindigkeit, die schnell genug ist, um zu verursachen, daß die genannten Gewebe-Klebstoff-Komponenten eine dünne Schicht auf mindestens einer inneren Oberfläche eines jeden Behälters bilden; und
(d) Gefrieren der genannten Gewebe-Klebstoff-Komponenten während der Rotation, so daß sie dünne Schichten aus gefrorenen Gewebe-Klebstoff-Komponenten (11, 21, 22, 31) bilden.

11. Verfahren nach Anspruch 10, bei welchem eine der Gewebe-Klebstoff-Komponenten Fibrinogen ist.

12. Verfahren nach Anspruch 10, bei welchem eine der Gewebe-Klebstoff-Komponenten Thrombin ist.

## Revendications

1. Système de distribution médicale destiné à rendre rapidement disponible des composants adhésifs tissulaires pour l'utilisation chirurgicale comprenant :
(a) un ou plusieurs récipients fermés (10, 20, 30), chacun des récipients fermés ayant un moins une surface intérieure ;
(b) un revêtement congelé mince d'un composant adhésif tissulaire (11, 21, 22, 31) sur au moins une surface intérieure de chacun des récipients fermés,
dans lequel le composant adhésif tissulaire demeure sous forme d'un revêtement congelé mince pendant le stockage mais est immédiatement décongelé avant administration pour utilisation chirurgicale.

2. Système de distribution médicale selon la revendication 1, dans lequel au moins l'un des composants adhésifs tissulaires comprend du fibrinogène.

3. Système de distribution médicale selon la revendication 1, dans lequel au moins l'un des composants adhésifs tissulaires comprend de la thrombine.

4. Système de distribution médicale selon la revendication 1, dans lequel au moins l'un des composants adhésifs tissulaires comprend un cryoprécipité de sang humain exempt de cellules.

5. Système de distribution médicale selon la revendication 1, dans lequel les récipients sont de forme cylindrique.

6. Système de distribution médicale selon la revendication 1, dans lequel le système de distribution médicale comprend des moyens permettant la décongélation rapide des composants adhésifs tissulaires congelés.

7. Système de distribution médicale selon la revendication 1, dans lequel les récipients comprennent des moyens pour administrer les composants adhésifs tissulaires après décongélation.

8. Système de distribution médicale selon la revendication 7, dans lequel les moyens de distribution comprennent une seringue (20).

9. Système de distribution médicale selon la revendication 7, dans lequel les moyens de distribution comprennent un distributeur aérosol (30).

10. Procédé pour la préparation d'un système de distribution médicale destiné à rendre des composants adhésifs tissulaires rapidement disponibles pour l'utilisation chirurgicale comprenant :
(a) l'introduction d'un ou de plusieurs composants adhésifs tissulaires à l'état liquide dans un ou plusieurs récipients (10, 20, 30), chaque récipient ayant au moins une surface intérieure ;
(b) la fermeture de ces récipients ;
(c) la rotation de ces récipients et des composants adhésifs tissulaires à une vitesse suffisamment élevée pour permettre aux composants adhésifs tissulaires de former un revêtement mince sur au moins une surface intérieure de chaque récipient; et
(d) congélation des composants adhésifs tissulaires pendant la rotation de façon à former des revêtements minces de composants adhésifs tissulaires congelés (11, 21, 22, 31).

11. Procédé selon la revendication 10, dans lequel l'un des composants adhésifs tissulaires est le fibrinogène.

12. Procédé selon la revendication 10, dans lequel l'un des composants adhésifs tissulaires est la thrombine.
